# EUROPEAN PATENT APPLICATION

(11) **EP 3 928 723 A1**
(43) Date of publication of application: **29.12.2021**
(21) Application number: 20182031.3
(22) Date of filing: 24.06.2020
(51) Int. Cl.: A61B 17/32, A61M 1/00

(54) **DEVICE, A CONTROL DEVICE, A SYSTEM AND A METHOD FOR LIPOSUCTION**

(71) Applicant: Avister-Medical, 4800 Verviers (BE)
(72) Inventor: DAUVISTER, Marc, 4800 Verviers (BE); DAUVISTER, Robert, 4860 Pepinster (BE)
(74) Representative: Calysta NV

(57) **Abstract**

Device for liposuction, a control device for controlling such a device for liposuction, a process for operating a device for liposuction and/or a system including the device for liposuction and the control device. The frequency and/or amplitude of an oscillation movement of a cannula caused by the device for liposuction is changeably by a control input.

## Description

### Technical Field

The present invention relates to a device, a control device, a system and a method for liposuction.

### Prior art

Liposuction is a type of fat removal procedure used in plastic surgery. The surgery can be performed minimal invasive by introducing a thin cannula into the relevant body region and drawing out the fat through the cannula introduced. Sometimes the removed material is re-injected in other parts of the body. This can be done for aesthetic reasons to increase certain parts of the body, but also for therapeutic reasons as the re-injected material proved to stimulate other cells to favour the cicatrisation. This effect depends however strongly on the amount of living stem cells in the extracted material.

The patent application EP1006895 discloses a liposuction system. A cannula is connectable to a handpiece. During the extraction, the cannula is vibrating in a back and forth movement (translational movement in axial direction) with full amplitude between the two turning points of the cannula being between 0,1-6 mm, preferably between 1-3 mm. The movement of the cannula in the handpiece is achieved with a pneumatique driving system.

Liposuction devices use sometimes ultrasonic vibrations of the cannula to improve the fat removal from the body. However, the ultrasonic vibrations caused severe damage in the tissue where the fat was removed. Therefore, it was suggested to use lower frequencies for the fat extraction.

The patent application US6336925 discloses to use such a liposuction device as explained above, however with a frequency between 10-500 Hz, preferably between 10-250 Hz and in one embodiment at around 15Hz and with a full amplitude of the cannula being between 2-10mm, preferably between 2-6 mm, more preferably between 3-5 mm. The movement of the cannula in the handpiece is achieved with a pneumatique driving system. The translational movement of the cannula in the axial direction causes also a radial translational movement perpendicular to the axial movement leading in their superposition to a nutation movement. This nutation movement was found to be very beneficial for the fat removal.

In the last decade, it was found out that body fat actually contains a high amount of stem cells. Stem cells deriving from fat material is also called adipose derived stem cells (ADSC).

EP2890310 discloses a liposuction system for ADSC with a high viability of stem cells. The described liposuction system works as the previous one described with a vibrational movement of the cannula in the axial direction with a frequency between 10-500 Hz, preferably between 10-50 Hz, more preferably between 15-20Hz and with a full amplitude of the cannula being between 1-10mm, preferably between 2-9 mm, more preferably between 5-8 mm. The movement of the cannula in the handpiece is achieved with a pneumatique driving system. It was found out that the fat extraction with this device increases the viability of the ADSC significantly compared to a manual extraction and provides a high percentage of stem cells with respect to the remaining material.

However, the described handpieces for liposuction have a plurality of disadvantages.

All liposuction handpieces use a pneumatique driving system. This requires an additional pneumatique connection to the handpiece and causes strong vibrations and shocks on the hand of the surgeon. In addition, the frequency and the amplitude of the pneumatique system is not controllable. The inventor found out that the frequency of the pneumatique system varied over time depending on temperature, aging of the material and further found out that the frequency of two equally manufactured pneumatique driving systems often varied significantly. Thus, the frequency and/or amplitude of pneumatique systems are first not precisely known and second can thus also not be controlled/varied by a user. The patent documents cited above mention as theoretical alternatives to the pneumatique drive an electric or magnetic driving system. These alternative drives were however never used as they overheat during the long liposuction surgeries which is very disturbing for the hand of the surgeon or do not provide sufficient oscillating power or require to much space for a handheld device. In addition, the requirements for the electro-magnetic radiation for medical equipment are strict and prevented the use of electric and magnetic driving systems.

### Brief summary of the invention

It is the object of the invention to provide an improved liposuction device, an improved control device and an improved system which avoid the problems of the state of the art, in particular which can comfortably be hold by the surgeon, which do not create damage to the patient and/or which improve the quality of the extracted material.

The object is solved by a device for liposuction, a control device for liposuction, a system for liposuction and a process for operating a device for liposuction according to the independent claims.

Changing the frequency and/or amplitude of the translational oscillation movement in a low frequency range allows to change between either an optimal frequency and/or amplitude for extracting ADSC with a high viability rate or an optimal frequency and/or amplitude for quickly removing the fat material from the body. When removing fat material from the body for retrieving ADSC, the quality of the ADSC is the most important point. The inventors found out that the correct frequency and/or amplitude is crucial for the viability of the ADSC. When removing the fat tissue, the most important point is a smooth and rapid removal of the fat tissue from the body to limit the impact of the surgery on the patient. Thus, by changing the frequency and/or amplitude, the oscillation movement of the cannula means can be optimized for the purpose of the surgery. This allows to increase the viability of stem cells for ADSC surgeries and to increase the velocity and throughput of pure fat extraction surgeries.

The object is further solved by a device for liposuction comprising a cannula means and a movement means configured to cause a translational oscillation movement of the cannula, characterized by one or more of the following embodiments.

The object is further solved by a control device for controlling a device for liposuction comprising a movement controller for controlling the movement of the cannula means of the device for liposuction, characterized by one or more of the following embodiments.

The object is further solved by a system comprising the device for liposuction and the control device, the system being characterized by one or more of the following embodiments.

The object is further solved by a process for operating a device for liposuction comprising the step of moving a cannula means of the device for liposuction by a translational oscillation movement of the cannula means. The process is characterized by one or more of the following embodiments.

The subsequent embodiments show optional features of the invention improving it further.

In one embodiment, the movement means is an electrical actuator configured to transfer electrical energy into the translational oscillation movement of the cannula means and/or the control signal for the device for liposuction is an electrical signal. The use of an electrical actuator for creating the translational oscillation movement of the cannula means in the device for liposuction instead of a pneumatique actuator allows to have a well-defined frequency for the oscillation movement which does not change over time and which could be controlled by the electric control signal. In addition, the electrical actuator reduces the vibrations, noise and shocks created by the pneumatique actuator of the state of the art. This significantly increases the comfort for the surgeon when operating the device for liposuction. Preferably, the movement means is an electro-magnetic actuator configured to cause the translational oscillation movement of the cannula means electro-magnetically. Preferably, the movement means is a linear electro-magnetic actuator.

In one embodiment, the oscillation movement of the cannula means has a frequency smaller than 100 Hz, preferably smaller than 50 Hz, preferably smaller than 30 Hz, preferably smaller than 25 Hz, preferably smaller or equal than 20 Hz. In one embodiment, the oscillation movement of the cannula means has a frequency larger than 5 Hz, preferably larger than 10 Hz, preferably larger than 12 Hz, preferably larger than 13 Hz, preferably larger than 14 Hz, preferably larger than or equal to 15 Hz. When the extraction of the fat is performed with oscillations in this frequency range, the percentage of living stem cells in the extracted material is significantly higher. In combination with an electrical actuator, the lower frequencies create less heat in the electrical actuator making the handling of the device for liposuction more comfortable for the surgeon. In addition, the electrical actuator can be precisely controlled in the frequency so that the surgeon knows exactly at which frequency he/she is operating.

In one embodiment, the oscillation movement of the cannula means has a full amplitude between the two turning points smaller than 15mm, preferably smaller than 14mm, preferably smaller than 13mm, preferably smaller than or equal to 12mm. In one embodiment, the oscillation movement of the cannula means has a full amplitude larger than 2mm, preferably larger than 3mm, preferably larger than 5mm, preferably larger than 6mm, preferably larger than 7mm, preferably larger than or equal to 8mm. When the extraction of the fat is performed with oscillations in this amplitude range, the percentage of living stem cells in the extracted material is significantly higher. In combination with the above mentioned frequencies, this provides an optimal range for the oscillation movement for the ADSC.

In one embodiment, the movement means comprises a first magnetic means (in the fixed portion) and a second magnetic means (in the movable portion), wherein one of the first and second magnetic means is an electro-magnetic means powered by electrical power and causing a magnetic field which interacts with the other of the first and second magnetic means to cause the translational oscillation movement of the movable portion and/or the cannula means. Preferably, the other of the first and second magnetic means is a permanent magnet. The permanent magnet is preferably arranged on the movable portion, preferably on the piston. Preferably, the permanent magnet has a cylindrical form, preferably a hollow cylindrical form. The permanent magnet has preferably a magnetic orientation between north pole and south pole which is coaxial or parallel to the longitudinal axis of the cannula means or the device for liposuction and/or to the translational movement of the oscillation of the cannula means. Preferably, the electro-magnetic means comprises at least one coil, preferably at least one solenoid. Preferably, the electromagnetic means comprises two coils, preferably two solenoids. Preferably, the two coils are arranged in the direction of the translational oscillation movement, of the longitudinal axis of the device or the cannula means or of the magnetic orientation of the permanent magnet at two sides of the permanent magnet and/or of the other of the first and second magnetic means. The electro-magnetic means is preferably arranged in the fixed portion. Preferably, the cylindrical permanent magnet is arranged around the piston. Preferably, the two coils are arranged (in the relaxed position of the permanent magnet) at the two ends of the permanent magnet and/or have a diameter equal or larger than the cylindrical permanent magnet (so that the permanent magnet can oscillate into the coils). The described realisation of the electro-magnetic actuator is particular advantageous for the present invention as it proved to show a high power and a low temperature. In addition, it does not need a classical electric motor which transfers a rotation into a translation which is less efficient.

In one embodiment, the device comprises a fixed portion and a movable portion. Preferably, the cannula means is part of the movable portion. Preferably, the movable portion is supported by the fixed portion movably in the direction of the longitudinal axis of the cannula means and/or of the device for liposuction. Preferably, the movable portion is a piston supported in a cylinder of the fixed portion. Preferably, a first end of the piston comprising the cannula means extends through the fixed portion outside of the cylinder and/or outside of the housing of the device. Preferably, the cylinder encloses the piston with the second magnetic means and/or the piston extending through the fixed portion outside of the cylinder is sealed with respect to the fixed portion in order to form a sealed cylinder.

In one embodiment, the device and/or the movement means and/or the electric control signal is not configured to make the cannula means rotate and/or the device comprises a blocking means configured to block a rotation of the cannula means. Preferably, the blocking means is realised by a form-fit of the fixed portion and of the movable portion which blocks the rotation of the movable part in the fixed part. It was found out that the rotation of the cannula can create severe damages for the muscles of the patient and does also increase the heat in the device for liposuction.

In one the movement means of the device for liposuction is configured to change the frequency and/or amplitude of the oscillation movement based on a control input. In one embodiment, the movement controller of the control device is configured to generate different control signals for generating the translational oscillation movement of the cannula means with different frequencies and/or amplitudes. In one embodiment, the frequency can be changed between at least two different frequency values and/or the amplitude can be changed between at least two different amplitude values. Preferably, the at least two different frequency values comprise at least one frequency value, preferably a plurality in the above mentioned frequency range, preferably between 10Hz and 25Hz, even more preferably between 15Hz and 20Hz. Preferably, the at least two amplitude frequency values comprise at least one amplitude value, preferably a plurality of amplitude values in the above mentioned full amplitude range, preferably between 2 and 15 mm, even more preferably between 8 and 12 mm.

In one embodiment, the control input is an electric control signal. The electric control signal is preferably generated in the control device. The electric control signal is preferably received at the device for liposuction from the control device. The electric control signal being configured for controlling the frequency and/or amplitude. The electric control signal being configured for powering the movement means. This allows to precisely control the optimal frequency and/or amplitude used for each application of the device for liposuction.

In one embodiment, the electric actuator or the movement means is electrically driven such that the movement of the movable portion is stopped electrically for moving in the opposed direction before stopping against the fixed portion. This avoids shocks which are unpleasant for the surgeon and which create fatigue for the device for liposuction.

In one embodiment, the cannula means is a fixture for a cannula for liposuction. The cannula can be fixed in the fixture. In another embodiment, the cannula means comprises the cannula as well.

In one embodiment, the device comprises a cannula which is connected to the cannula means or which is part of the cannula means.

In one embodiment, the device for liposuction is a handheld device.

In one embodiment, the device comprises a first portion and a second portion, wherein the first portion comprises the movement means and the cannula means, wherein the second portion comprises the handle of the device. Preferably, the handle is configured to grab the device with a hand and/or to guide the device and the cannula means during operation. This has the advantage that the heat of the movement device does not disturb the hand of the surgeon and/or the heat of the movement device can be dissipated better. This allows further to have a movement device larger than the diameter for a handle. Preferably, the first portion is arranged at a first end of the device in the direction of the longitudinal axis of the cannula means and/or of the device, and the second portion is arranged at a second end of the device opposed to the first end of the device. Preferably, the diameter of the first portion is larger than the diameter of the second portion.

In one embodiment, the movement controller is configured to control the frequency and/or amplitude of the movement of the cannula means of the device for liposuction based on the generated electric control signal.

In one embodiment, the control device comprises a pressure means connected to a cannula of the device for liposuction and configured to create an depression for drawing fat into the cannula and/or to create a pressure for injecting an injection material out of the cannula into a patient. Preferably, the pressure means comprises a first pressure means, preferably a vacuum pump for creating a depression to draw material into the cannula of the device. In one embodiment, the pressure means comprises additionally to the first pressure means a second pressure means (distinct from the first pressure means) for injecting something (e.g. physiological liquid or extracted fat) through the cannula into the body of the patient. In another embodiment, the same first pressure means is used to create the pressure for the injection. In another embodiment, the control device is not configured to create a pressure for injection. In one embodiment, the value of the pressure and/or the depression can be controlled, preferably by a user interface.

In one embodiment, the control device comprises a user interface for inputting the frequency and/or amplitude of the translational oscillation movement of the cannula means and/or switching on or off the translational oscillation movement of the cannula means and/or inputting the value of the depression or the pressure of the pressure means and/or switching on or off the pressure means and/or for giving out the frequency and/or amplitude of the translational oscillation movement of the cannula means and/or for giving out the pressure and/or depression of the pressure means. Preferably, the user interface comprises a display for giving out the mentioned information. Preferably, the display is a touch screen for inputting any of the above-mentioned information, e.g. the frequency of the cannula means. Preferably, the user interface comprises further a pedal for controlling the operation of the device for liposuction. Preferably, the pedal switches on and off the movement means and even more preferably controls the amplitude of the oscillation movement. Preferably, the pedal switches on and off the pressure means, in particular the first pressure means and even more preferably controls the value of the depression created by the first pressure means. Preferably, the pedal controls the movement means of the device and the (first) pressure means connected to the cannula of the device in a synchronized way.

In one embodiment, the control device or the movement controller is configured to control two or more devices for liposuction and/or the control output is configured for connecting two (or more) devices for liposuction and/or the user interface is configured to connect two or more external user input devices, in particular two or more pedals. The surgery for fat extraction is long. In university hospitals, the surgery is often performed by two surgeons requiring to systems for liposuction which is expansive and requires more space. By controlling with the same control device two devices for liposuction, this problem is overcome.

In one embodiment, the movement controller, the control output and the pressure means are arranged in the same device. Preferably, the control device comprises a housing for inserting a movement control module and/or for inserting a pressure module and/or for inserting at least one container for the extracted fat and/or for fat to be injected and/or for physiological liquid to be injected. The movement control module comprises the movement controller, the control output and/or the user interface, in particular the touch screen or display. The pressure module comprises the pressure means. The control of the pressure means can be arranged in the pressure module and/or in the movement control module. Preferably, the control of the pressure means is arranged in the pressure module such that the pressure module could work as a stand-alone device, but can be controlled as well by the movement control module.

In one embodiment, a modular set for mounting the control device in different configurations is protected. The modular set comprises a modular housing for housing at least two modules. The at least two modules comprise the movement control module and the pressure module. The modular housing comprises preferably a movement control module spot for housing the movement control module. The modular housing comprises preferably a pressure module spot for housing the pressure module. Preferably, the set comprises at least two different types of movement control modules. A first type of movement control module is configured to control and/or connect (only) one device for liposuction. A second type of movement control module is configured to control and/or connect two devices for liposuction. Preferably, the set comprises at least two types of pressure module. A first type of pressure module is configured to create only a depression at the cannula for extracting material through the cannula. A second type of pressure module is configured to create only a depression at the cannula for extracting material through the cannula and to create an overpressure at the cannula to inject material through the cannula 210. A third and fourth type of pressure module are as the first and second type of pressure modules, respectively, but configured to provide the respective pressure for two devices for liposuction. Preferably, the control device can be made modularly from one or more or combinations of the following configurations: A first configuration with a movement control module (and no pressure module); a second configuration with a movement control module and a pressure module; a third configuration with the first type of the movement control module (and no or one pressure module); a fourth configuration with the second type of the movement control module (and no or one pressure module); a fifth configuration with the first type of the pressure module; a sixth configuration with the second type of the pressure module; a seventh configuration with the third type of the pressure module.

In one embodiment, the device for liposuction is a distinct device as the control device. Preferably, the device for liposuction is connected to the control device via a wire for powering the movement means and/or for controlling the movement device. However, it is also possible that the device for liposuction comprises a battery or any other energy source. This would allow to transfer the electric control signal from the control device to the device wirelessly. In one embodiment, the device for liposuction is connected to the control device wirelessly for controlling the movement device.

In one embodiment, the control device and the device for liposuction are arranged in the same device, preferably the handpiece.

### Brief description of the Drawings

Figure 1 is an embodiment of a system for liposuction.
Figure 2 is an embodiment of a device for liposuction.

In the drawings, the same reference numbers have been allocated to the same or analogue element.

### Detailed description of an embodiment of the invention

Other characteristics and advantages of the present invention will be derived from the non-limitative following description, and by making reference to the drawings and the examples.

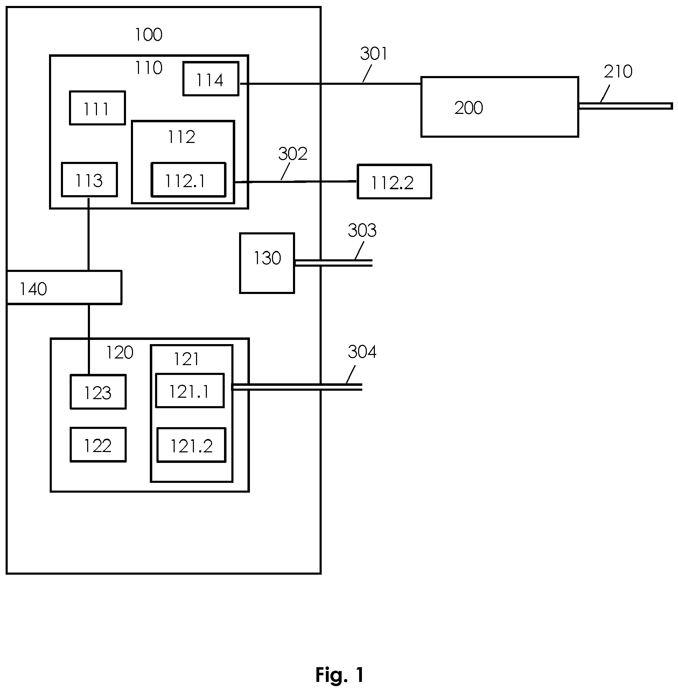

Fig. 1 shows an embodiment of a system for liposuction. The system comprises a control device 100 and a device for liposuction 200.

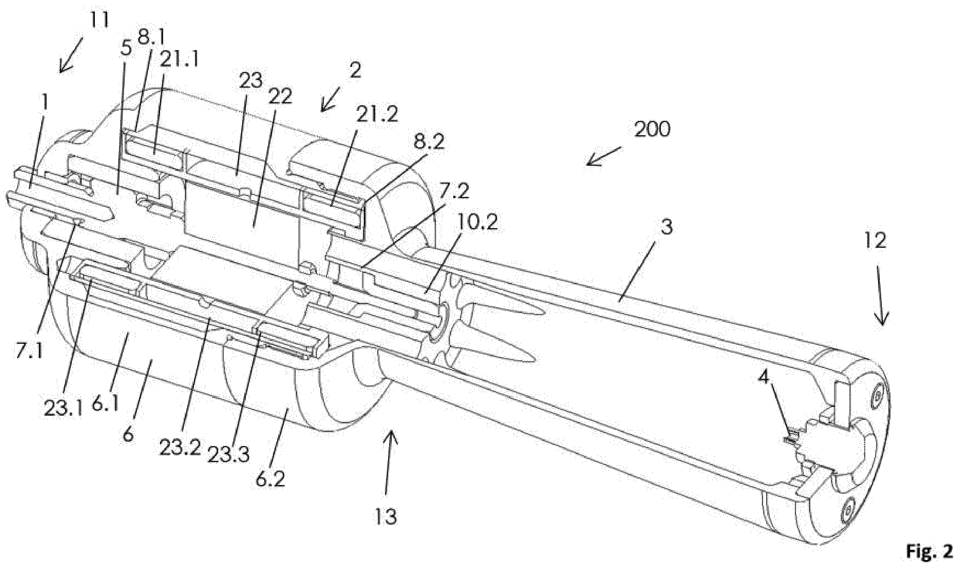

Fig 2 shows an embodiment of the device for liposuction 200.

The device for liposuction 200 is preferably a handpiece, i.e. a device which can be held by a human hand and which can be guided manually. Obviously, a handpiece could also be operated by a robot or a machine. However, the device for liposuction could be also different than a handpiece, e.g. the end for an robot arm or a tool for a surgery machine.

The device for liposuction 200 comprises a cannula means 1 and a movement means 2. The device for liposuction 200 comprises preferably a housing 6, a handle 3, a control interface 4, a piston 5 and/or a guide means 7.

The device for liposuction 200 comprises preferably a longitudinal axis extending in a first direction or also called longitudinal direction. The device for liposuction comprises preferably a second direction or radial direction extending perpendicular or radially to the longitudinal direction. The device for liposuction 200 comprises in the longitudinal direction a first end 11 and a second end 12, wherein the second end 12 is opposed to the first end 11. The device for liposuction 200 comprises preferably a first portion and a second portion. The first portion is arranged between the first end 11 and a point 13 arranged between the first end 11 and the second end 12. The second portion is arranged between the second end 12 and the point 13 arranged between the first end 11 and the second end 12. The device for liposuction 200 comprises preferably a movement direction. The movement direction is preferably parallel to the longitudinal direction, but could extend also in another direction.

Preferably, the device for liposuction 200 is a handpiece. The device for liposuction 200 comprises preferably a handle 3. The handle 3 is configured to hold the device for liposuction 200 in a hand (of a surgeon) and/or to guide the device for liposuction 200 (during a surgery). The handle 3 is preferably arranged in the second portion of the device for liposuction 200. The handle 3 has preferably the form of a cylinder or conical frustum. In the latter case, the handle tapers preferably towards the point 13 between the first end 11 and the second end 12.

Preferably, the device for liposuction 200 comprises a movable portion and a fixed portion.

The movable portion is supported movably in the fixed portion. The movable portion is supported movably in the movement direction, preferably in the longitudinal direction. The movement direction has two opposed directions, a first movement direction and a second movement direction. The first and second movement directions are parallel, but have opposed directions. The movable portion is arranged to perform an oscillation movement. The oscillation movement is an oscillation in the movement direction. The oscillation movement describes a translational movement in the movement direction. The movable portion does preferably not provide or blocks a rotational movement around the movement direction and/or does not provide or blocks a translational movement/component perpendicular to the movement direction. In a less preferred embodiment, it is also possible that the movable portion performs in the operation a movement comprising a superposition of the of the oscillation movement of the movable portion in the movement direction with a further movement, e.g. a rotation, e.g. a rotation of the movable portion around the movement direction, and/or with a further translation, e.g. a radial translational movement of the movable portion perpendicular to the movement direction. However, the subsequent description of the oscillation movement refers just to the movement in the movement direction. The movable portion is supported such that it moves along a movement axis, preferably the longitudinal axis of the device for liposuction 200. The oscillation movement comprises an oscillation around a rest position. The oscillation movement comprises periodically a movement from the rest position in the first movement direction up to a first turning position and then in the second movement direction from the first turning position through the rest position to a second turning position where the movable portion turns again to move back in the first movement direction to the rest position.

This oscillation movement is repeated periodically with an oscillation frequency. The oscillation frequency is preferably lower than 100 Hz, preferably lower than 50 Hz, preferably lower than 40 Hz, preferably lower than 30 Hz, preferably lower than 25 Hz, preferably lower or equal than 20 Hz. The oscillation frequency is preferably larger than 1 Hz, preferably than 5 Hz, preferably than 7 Hz, preferably than 9 Hz. Preferably, preferably the oscillation frequency is between 10Hz and 25 Hz, preferably between 15Hz and 20Hz. The movable portion comprises preferably the cannula means 1. The movable portion comprises preferably the piston 5 and the movable parts of the movement means 2. The piston 5 is preferably arranged coaxially with the movement axis, preferably the longitudinal axis of the device for liposuction 200. In a preferred embodiment, the frequency of the oscillation movement can be changed (by a user or by the control device or by the movement means 2) between at least a minimum frequency and a maximum frequency. This means that the handpiece can be operated with at least two frequencies, at least with the minimum frequency and the maximum frequency. Preferably, the at least two frequencies comprise the minimum frequency, the maximum frequency and a plurality of frequencies inbetween. The plurality of frequencies could be a plurality of discrete and equidistant frequency values between the minimum and maximum frequency or could also comprise a continuum of frequency values between the minimum and maximum frequency. The maximum frequency of the oscillation movement is preferably lower than 100 Hz, preferably lower than 50 Hz, preferably lower than 40 Hz, preferably lower than 30 Hz, preferably lower than 25 Hz, preferably lower or equal than 20 Hz and/or preferably larger than 18 Hz, preferably than 19 Hz, preferably than 20 Hz, maybe than 25 Hz. The minimum frequency of the oscillation movement is preferably larger than 1 Hz, preferably than 5 Hz, preferably than 7 Hz, preferably than 9 Hz, preferably than 10 Hz, preferably than preferably than 12 Hz, preferably than 13 Hz, preferably than 14 Hz, preferably than or equal to 15 Hz and/or lower than 17 Hz, preferably lower than 16 Hz, preferably lower or equal than 15 Hz. In a preferred embodiment, the frequency can be changed between the minimum frequency of 15Hz and the maximum frequency of 20 Hz. In another embodiment, it is also possible that the frequency of the oscillation movement is fixed and not controllable by a user.

In the field of liposuction devices, the term amplitude is often used for the full amplitude of the oscillation movement indicating the displacement of the movable portion or the cannula means during the oscillation movement between the two turning positions of the oscillation movement, i.e. between the two most distal location of one point of the cannula means or the movable portion during the oscillation movement. The half amplitude of the oscillation movement indicates the displacement of the movable portion or the cannula means during the oscillation movement from the rest position in the first or second movement direction. The full amplitude corresponds to twice the half amplitude. The full amplitude of the oscillation movement is preferably larger than 1 mm, preferably than 2 mm, preferably than 3 mm, preferably than 4 mm, preferably than 5 mm, preferably than 6 mm, preferably than 7 mm. The full amplitude of the oscillation movement is preferably smaller than 20 mm, preferably than 15 mm, preferably than 14 mm, preferably than 13 mm, preferably smaller than or equal to 12 mm. Thus, the movable portion moves in the movement direction the full amplitude from one turning position to the other turning position. The full amplitude of the oscillation movement is limited by a maximum physical amplitude which is defined by the maximum movement possible in the fixed portion. Preferably, the maximum physical amplitude is defined by a physical stop, preferably two physical stop positions in which the movable portion stops against the fixed portion. Preferably, the full amplitude of the oscillation movement is defined by the movement means 2, not by the physical stop position(s). That is that the two turning positions are distinct from the physical stop positions. This avoids shocks, when the movable portion hits against the physical stop position(s). In this document, if the distinction between full amplitude and half amplitude is not important, just the term amplitude will be used. In doubt, the term amplitude shall indicate the full amplitude. In a preferred embodiment, the (full/half) amplitude of the oscillation movement can be changed (by a user or by the control device or by the movement means 2) between at least a minimum amplitude and a maximum amplitude. This means that the handpiece can be operated with at least two amplitudes, the minimum amplitude and the maximum amplitude. Preferably, the at least two amplitudes comprise the minimum amplitude, the maximum amplitude and a plurality of amplitudes between the minimum and maximum amplitude. The plurality of amplitudes could be a plurality of discrete and equidistant amplitude values between the minimum and maximum amplitude or could also comprise a continuum of amplitude values between the minimum and maximum amplitude. The maximum full amplitude of the oscillation movement is preferably larger than 10 mm, preferably larger than 11 mm, preferably larger or equal to 12mm. The maximum full amplitude of the oscillation movement is preferably smaller than 20 mm, preferably than 15 mm, preferably than 14 mm, preferably than 13 mm. The minimum full amplitude of the oscillation movement is preferably larger 1 mm, preferably than 2 mm, preferably than 3 mm, preferably than 4 mm, preferably than 5 mm, preferably than 6 mm, preferably than 7 mm, preferably larger than or equal to 8 mm. The minimum full amplitude of the oscillation movement is preferably smaller than 10 mm, preferably than 9 mm, preferably than 8 mm. In another embodiment, it is also possible that the amplitude of the oscillation movement is fixed and not controllable by a user.

The oscillation movement was described here for the movable portion. The description of the oscillation movement of the movable portion holds equally for the oscillation movement of the cannula means 1, the cannula 210, the piston 5 and/or the second magnetic means and is not repeated for those for the sake of brevity. The oscillation movement of the cannula means 1 transfers the oscillation movement to the cannula 210 so that the cannula 210 performs this oscillation movement in the axial direction of the cannula 210. Due to the flexibility of the cannula 210 and other external forces like gravity and others, the cannula 210 performs a superposition of the oscillation movement in the movement direction or the axial direction of the cannula 210 and of a radial oscillation movement perpendicular to the axial direction of the cannula 210. This superposition of the oscillation movement and the radial oscillation movement is also called nutation movement as explained above. The radial oscillation movement is preferably a translational movement in a radial direction (any direction perpendicular to the axial direction of the cannula 210), wherein the radial direction rotates around the axial direction. However, the cannula 210 itself does not rotate around the axial direction of the cannula 210. There may be an embodiment, in which the movable portion of the handpiece rotates to create the oscillation movement. However, such a rotation of the movable portion could not be transmitted to the cannula 210. The frequency and/or amplitude of the radial oscillation movement for a given oscillation movement is preferably defined by the length and/or flexibility of the cannula 210.

The fixed portion supports the movable portion. The fixed portion comprises preferably the housing 6, the guide means 7, the sealings 10 and/or fixed parts of the movement means 2.

The cannula means 1 is preferably a fixture for fixing a cannula 210 (not shown in Fig. 1). The fixture is preferably a thread, preferably a female thread. However, other fixture mechanisms are also possible. The cannula 210 is preferably disposable, i.e. is used only for one surgery and/or for one patient. The device for liposuction 200 comprises preferably as well the cannula 210 connected to the fixture 1. The cannula 210 has a proximal end connected to the fixture 1 and a distal end configured for inserting in a body portion of a patient. The cannula 210 comprises preferably a cannula fixture to be connected to the fixture of the cannula means 1, e.g. a thread, preferably a male thread. The body portion is preferably the region of the body from which fat should be extracted. The cannula 210 has preferably a longitudinal axis extending between the proximal end and the distal end. The longitudinal axis is preferably parallel to the longitudinal direction of the device for liposuction 200 and/or to the movement direction and/or coaxial to the longitudinal axis of the device for liposuction 200. The cannula 210 comprises a channel with a first opening at the distal end of the cannula 210 and with a second opening to be connected to a pressure means 120 and/or a container 130 described in more detail below. The channel connects the first and second opening. The channel extends preferably along the longitudinal axis of the cannula 210. The cannula means 1 has preferably also a longitudinal axis which is preferably parallel to the longitudinal axis of the cannula 210 and/or to the longitudinal direction or the movement direction of the device for liposuction 200. The longitudinal axis of the cannula means 1 is preferably the thread axis of the fixture. In another embodiment, the cannula means 1 could include directly the cannula 210.

The movement means 2 is configured to drive or generate the oscillation movement of the movable portion and/or of the cannula means 1 described above. The movement means 2 is preferably configured to drive the movable portion to perform the oscillation movement. The movement means 2 is preferably configured to drive the movable portion to perform a translational oscillation movement, preferably a pure translational oscillation and/or without a rotation of the movable portion. However, in a less preferred embodiment, it is also possible that the oscillation movement creates a rotation or other movement superposed on the translational oscillation movement.

The movement means 2 preferably drives directly the movable portion and/or causes directly the oscillation movement described above. This is more efficient and reduces space and heat in the device for liposuction 200. However, in a less preferred embodiment, it is also possible that the movement means 2 creates the oscillation movement of the movable portion indirectly. Creating the oscillation movement of the movable portion indirectly means that the movement means 2 creates an intermediate movement of an intermediate movable portion which creates through a movement conversion means the oscillation movement of the movable portion. For example, the intermediate movable portion could be driven by the movement means 2 to perform a (pure) rotation which is transferred over a cam surface or a crank shaft as a movement conversion means to a (pure or non-pure) translational movement of the movable portion.

The movement means 2 is preferably an electric actuator. The electric actuator is any means which converts electrical energy and/or an electric control signal received in the device for liposuction 200 into the oscillation movement (directly or indirectly). The electric actuator has the advantage over the pneumatique actuator that the frequency of the movable portion can be precisely set and/or controlled. In addition, electric actuators create less vibrations and shocks than pneumatique actuators which makes the handling of the device for liposuction 200 much more comfortable for the surgeon. In a preferred embodiment, the electric actuator is an electro-magnetic actuator. An electro-magnetic actuator converts the electrical energy and/or the electric control signal in the device for liposuction 200 into a magnetic field which creates/causes the oscillation movement (directly or indirectly). However, other embodiments of an electric actuator are also possible e.g. a piezo-electric actuator. Subsequently, a preferred embodiment of an electro-magnetic actuator will be described.

The electro-magnetic actuator (or the movement means 2) comprises a first magnetic means arranged in the fixed portion and a second magnetic means arranged in the movable portion (including here also the meaning that the second magnetic means is included in the intermediate movable portion). The first magnetic means is preferably an electro-magnetic means for converting the electric energy into a magnetic field causing a movement, preferably the oscillation movement or less preferably the intermediate movement, of the second magnetic means or the movable portion. The second magnetic means is preferably a permanent magnet 22. In a less preferred embodiment, the second magnetic means could also be an electro-magnetic means as described for the first magnetic means. In a less preferred embodiment, the electro-magnetic means as the second magnetic means could be arranged in the movable portion and the permanent magnet 22 as first magnetic means in the fixed portion. The second magnetic means is preferably arranged in the movable portion for creating directly the oscillation movement. However, it is also possible that the second magnetic means is connected to the movable portion to create the oscillation movement only indirectly as described above.

The permanent magnet 22 is preferably arranged on the piston 5. The permanent magnet 22 has preferably a magnetic orientation parallel to the movement direction and/or parallel to the magnetic field created by the first magnetic means or parallel to the coil axis of the latter. The magnetic orientation is preferably the magnetic line between the north pole and the south pole of the permanent magnet. The permanent magnet 22 is a simple dipole arrangement with one magnetic orientation. The magnetic orientation of the permanent magnet 22 can be different or more complex depending on the arrangement of the electromagnetic means. The permanent magnet 22 has preferably a cylindrical form, preferably a circular cylindrical form. Preferably, the magnetic orientation is parallel to the cylinder axis of the permanent magnet 22. The permanent magnet 22 has preferably an outer diameter D2 (in the second or radial direction) and/or a length L1 (in the longitudinal direction or the movement direction). The permanent magnet 22 is preferably guided in a (hollow) cylinder provided by the fixed portion such that the permanent magnet 22 can move in the movement direction in the cylinder of the fixed portion. Preferably, the permanent magnet 22 is fixed on the movable portion, preferably the piston 5 such that the permanent magnet 22 cannot move in the movement direction with respect to the movable portion or the piston 5, preferably that that the permanent magnet 22 cannot move at all with respect to the movable portion or the piston 5. In a preferred embodiment, the permanent magnet 22 has the form of a hollow cylinder, wherein the piston 5 extends through the inner opening of the hollow cylindric permanent magnet 22. Preferably, the permanent magnet 22 is fixed on the piston 5 by a stop or a portion with a larger diameter than the opening of the hollow cylinder of the permanent magnet 22 arranged on both sides of the permanent magnet 22. Preferably, the piston 5 comprises two piston portions which can be fixed to each other and between which the permanent magnet 22 will be arranged. Each piston portion comprises one of the mentioned two stops of the piston 5. Preferably, the two piston portions can be screwed to each other and pressing so the permanent magnet 22 between the two stops. Here the second piston portion is just a screw nut. However, it is also possible to fix the permanent magnet 22 differently on the piston 5. For example, the permanent magnet 22 can be realised as a full cylinder and/or the piston 5 could be fixed to the base surface of the permanent magnet 22 (pointing towards the first end 11 of the device for liposuction 200). The permanent magnet 22 comprises in the movement direction or the direction of the cylinder axis a first end and a second end opposed to the first end. The permanent magnet 22 is preferably made of Neodyme (NdFeB). However, other materials for the permanent magnet 22 are possible. Preferably, the permanent magnet 22 is coated by a (thin) nickel layer, preferably (Ni-Cu-Ni) to make the permanent magnet 22 harder and to protect the permanent magnet 22.

The electro-magnetic means (in the first magnetic means) comprises preferably at least one coil 21, preferably two coils 21.1, 21.2. The at least one coil 21 is preferably at least one solenoid or the two coils are two solenoids. The coil axis (around which the at least one coil is wound or arranged) is preferably parallel, coaxial or identical to the movement direction/axis or the longitudinal direction/axis of the device for liposuction 200 or to the magnetic orientation of the second magnetic means or to the cylindrical axis of the permanent magnet 22. The at least one coil 21 is or the two coils 21.1, 21.2 are arranged such that the permanent magnet 22 can move within the at least one, preferably two coil(s) 21.1, 21.2. The at least one coil 21 has or the two coils 21.1, 21.2 have an inner diameter D1 larger than the outer diameter D2 of the permanent magnet 22 so that the permanent magnet 22 can move inside the at least one coil 21 or inside the two coils 21.1, 21.2. The inner diameter D1 is preferably substantially the same as the outer diameter D2 so that the gap between the permanent magnet 22 and the at least one, preferably two coil(s) 21.1, 21.2 is very small. Preferably, the two coils comprise a first coil 21.1 and a second coil 21.2. The first coil 21.1 is arranged in the movement direction at the first end of the permanent magnet 22 in its rest position. Preferably, the first coil 21.1 extends in the movement direction from the first end of the permanent magnet 22 in its rest position towards the first end 11 of the device 100 or away from the permanent magnet 22. The second coil 21.2 is arranged in the movement direction at the second end of the permanent magnet 22 in its rest position. Preferably, the second coil 21.2 extends in the movement direction from the second end of the permanent magnet 22 in its rest position towards the second end 12 of the device 100 or away from the permanent magnet 22. The first coil 21.1 and/or the second coil 21.2 have in the movement direction a length L2. The length L2 of the first and/or second coil 21.1, 21.2 is preferably smaller than the length of the permanent magnet L1, preferably smaller than 0.8^{∗}L1 (0.8 times LI), preferably smaller than 0.7^{∗}L1, preferably smaller than 0.6^{∗}L1. The length L2 is preferably larger than 0.2^{∗}L1 preferably larger than 0.3^{∗}L1, preferably larger than 0.4^{∗}L1. Preferably, the length L2 is roughly half of the length L1. Preferably, the length L2 is larger than the maximal amplitude or the maximum physical amplitude of the movable portion or the permanent magnet 22, preferably larger than twice the maximal amplitude or the maximum physical amplitude of the movable portion or the permanent magnet 22. The two coils are arranged in the movement direction in a distance of at least 0.5^{∗}L1, preferably of at least 0.7^{∗}L1, of at least 0.8^{∗}L1, preferably of at least 0.9^{∗}L1. The two coils are arranged in the movement direction in a distance of less than 1.5^{∗}L1, preferably of less than 1.3^{∗}L1, of less than 1.2^{∗}L1, preferably of less than 1.1^{∗}L1. Preferably, the two coils 21.1 and 21.2 are arranged in a distance of L1. The shown embodiment comprises a coil 21.1, 21.2 at each end of the permanent magnet 22 which is preferred as it increases the power of the electro-magnetic actuator. However, it would also be possible to work with only one of the two coils 21.1, 21.2 or with one coil being arranged at the same height as the permanent magnet 22 or other arrangements of the coils. The at least one coil 21 is preferably a copper coil. However, other materials are also possible for the coil(s) 21. The at least one coil(s) 21 is/are preferably enamelled. The at least one coil 21 is wound or arranged in a bobbin. Preferably, the bobbin 23 comprises two coil sections 23.1 and 23.2 for winding or arranging the first coil 21.1 and the second coil 21.2. Preferably, the same bobbin 23 is used for the two coils 21.1 and 21.2. Preferably, the bobbin 23 comprises a distancing section 23.3 between the two coil sections 23.1 and 23.2. The bobbin 23 with the two coils 21.1 and 21.2 form preferably a sleeve or a hollow cylinder. The inner wall of the bobbin 23 preferably guides the permanent magnet 22 along the movement direction. The bobbin is preferably longer than the length L1 of the permanent magnet 22 plus twice the maximum physical amplitude of the movable portion or the permanent magnet. This allows that the bobbin extends beyond the stops for the movable portion or the permanent magnet 22.

The at least one coil, preferably the two coils 21.1, 21.2 is/are controlled such that the permanent magnet 22 oscillates in the movement direction so that the movable portion performs the oscillation movement. The at least one coil, preferably the two coils 21.1, 21.2 are controlled by an electric control signal received at the electrical control interface 4 of the device for liposuction 200. The electric control signal is preferably a periodical signal with the desired frequency of the oscillation movement. The electric control signal could be a sinusoidal signal, an on-off signal or a pulse-width-signal (PWM) signal. The electric control signal is preferably a two-phase signal, with a first phase signal for the first coil 21.1 and a second phase signal for the second coil 21.2. The second phase signal corresponds preferably to the first phase signal with phase shift of 180°. The electric control signal is preferably configured to control the frequency and/or amplitude of the oscillation movement. The amplitude of the oscillation movement corresponds to the intensity of the force of the movable portion. The electric signal is preferably configured to transfer the electric control signal and the power for the oscillation movement. However, it is also possible, that the electrical control signal is a pure control signal and the power for the oscillation movement comes from a battery or other energy source in the device for liposuction 200. The electric control signal has preferably a voltage equal or lower than 24 Volt (V). The electric interface is preferably a cable connected to the control device 100 or a socket for connecting a cable to the control device 100. However, it is also possible that the electric interface 4 is a wireless interface for receiving the electric control signal from the control device 100 wirelessly. The electrical interface 4 is connected with the at least one, preferably the two coil(s) 21.1, 21.2 (connection not shown in Fig. 1). The electric interface 4 is preferably arranged at the second end 4 of the device for liposuction 200.

One embodiment of the electro-magnetic actuator was described. The electromagnetic actuator can also be arranged differently. The electro-magnetic actuator can also comprise only one coil which drives the permanent magnet against the force of a spring. The electro-magnetic actuator can also be realized as linear electro-magnetic motor or as rotating electro-magnetic motor whose rotation movement is transformed in a translational oscillation movement. The movement means 2 was described to drive the movable portion. The description of driving the movable portion shall equally apply to driving the cannula means 1, the piston 5 and or the first magnetic means.

The movement means 2 is preferably arranged in the first portion of the device for liposuction 200. The first portion has preferably a larger thickness/diameter (in the second or radial direction) than the second portion. The separation of the handle 3 in the second portion and the movement means 2 in the first portion has several advantageous. It allows to make the first portion thicker than the handle which allows to realize more power in the movement means and a more comfortable handle optimized by ergonomic and not electrical constraints. In addition, the heat created by the movement means 2 can dissipate better which increases the efficiency of the movement means 2, and/or the heat of the movement means 2 does not heat the handle 3 which could be uncomfortable for the surgeon for longer surgeries. In addition, the step created by the transition from the second portion to the first portion provides an ergonomic stop for the hand holding the handle 3 which further improves the guidance of the device for liposuction 200.

The guide means 7 are configured to guide the movable portion or the piston 5 along the movement direction. The guide means 7 comprises preferably a first guide means 7.1 and a second guide means 7.2. The first guide means 7.1 guides the movable portion or the piston 5 on a first side of the second magnetic means or the permanent magnet 22. The second guide means 7.2 guides the movable portion or the piston 5 on a second side of the second magnetic means or the permanent magnet 22. The first and/or second guide means 7.1, 7.2 are preferably sleeves. However, other guide means are also possible. The guide means 7 are preferably further configured to block any radial movement of the movable portion and/or to block any rotation of the movable portion. This can be achieved for example by a form-fit between the movable portion and the fixed portion which allows the movement in the movement direction, but blocks a rotation of the movable portion with respect to the fixed portion. The form-fit can for example be achieved by a groove in one of the guide means 7 (preferably the first guide means 7.1) and the piston 5 and by a projection of the other of the guide means 7 (preferably the first guide means 7.1) and the piston 5 extending in the groove.

The housing 6 encloses preferably the movement means 2 and the piston 5. The housing 6 or the fixed portion comprises an opening through which the movable portion or piston 5 with the cannula means 1 extends. The opening is preferably arranged coaxially to the movement axis, preferably the longitudinal axis of the device for liposuction 200. The movable portion / piston 5 in operation oscillates in the opening of the fixed portion or housing 6. The movable portion / piston 5 is preferably sealed with respect to the opening in the housing 6 or the fixed portion so that the inside of the housing 6, in particular the movement means 2 is sealed with respect to the outside. This is achieved preferably by a first sealing 10.1. The sealing is preferably arranged in the opening of the housing 6, but could be also arranged somewhere else. The sealing is preferably configured to hold back hot steam from sterilization devices, i.e. humid heat at a temperature of more than 100°C, preferably more than 110°C, preferably morthan 120°C, preferably more than 130°C and at a pressure of more than 1 Bar, preferably more than 2 Bar, preferably at 3 Bar or more. The device for liposuction comprises preferably a second sealing 10.2 which seals the piston 5 / movable portion on its second end (opposed to the cannula means 1). The second sealing 10.2 is preferably arranged in the second guide means 7.2. Preferably, the second sealing 10.2 has the function of sealing and guiding the piston 5.

The housing 6 comprises preferably (at least around the movement means 2) a material which blocks the magnetic field from the movement means 2 or the electro-magnetic actuator or the at least one coil 21. Preferably, the material of the housing blocking the magnetic field is a paramagnetic, ferromagnetic or ferrite material. Preferably, a ferrite material is used. Ferrite material is preferably a ceramic material made by mixing and firing large proportions of iron (III) oxide (Fe2O3) blended with small portions of one or more additional metallic elements such as barium, manganese, nickel, zinc or others. Ferrite material is non-conductive and ferrimagnetic. Preferably, the complete housing is made of this magnetic field blocking material to have no magnetic exposure of the surgeon or the patient. The piston 5 on the other side comprises preferably an amagnetic material (with a low magnetic permeability) so that the electric field created by the permanent magnet and the electro-magnetic means is not leaving the device 200 where the piston 5 extends through the housing 6.

The housing 6 comprises preferably a first housing part 6.1 and a second housing part 6.2. The first housing part 6.1 covers the first end 11 of the device 200 and/or at least a portion of the first portion of the device 200. The second housing part 6.2 covers the second end 12 of the device 200 or the second portion and a part of the first portion of the device 200. The first housing part 6.1 includes or encloses preferably the first guide means 7.1, the bobbin 23 and/or the opening for the movable portion or the piston 5. The second housing part 6.2 includes or encloses the handle 6, the electric interface 4, the second guide means 7.2. The first housing 6.1 comprises preferably a first support recess 8.1 for supporting a first end of the bobbin 23 and/or the first coil 21.1. The second housing 6.2 comprises preferably a second support recess 8.2 for supporting a second end of the bobbin 23 and/or the second coil 21.2. The first and/or second support recess 8.1, 8.2 are preferably arranged coaxially to the piston 5, the longitudinal axis or movement axis of the device 200 and/or the permanent magnet 22. For mounting the device for liposuction 200, the piston 5 with the permanent magnet 22 is inserted with the end of the piston 5 with the cannula means 1 (from the inside of the housing 6) through the opening in the first housing part 6.1. Preferably, the piston 5 is inserted with the cannula means 1 through the first guide means 7.1 and the opening, wherein the first guide means 7.1 is mounted before the inserting step. The bobbin 23 with the coils 21.1, 21.2 is inserted in the first housing part 6.1, preferably in the first support recess 8.1. The second housing part 6.2 is closed on the first housing part 6.1 so that the (second end of the) bobbin 23 is supported in the second support recess 8.2 and/or that the (second end of the) piston 5 is supported in the second guide means 7.2. The first housing part 6.1 and the second housing part 6.2 are preferably closed by a thread connection. The first housing part 6.1 and the second housing part 6.2 are preferably closed in a sealed way, e.g. by a sealing. The sealing is preferably configured to hold back hot steam from sterilization devices, i.e. humid heat at a temperature of more than 100°C, preferably more than 110°C, preferably mor than 120°C, preferably more than 130°C and at a pressure of more than 1 Bar, preferably more than 2 Bar, preferably at 3 Bar or more.

The control device 100 comprises a movement controller 111 and a control output 114. The control device 100 comprises preferably a user interface 112, a power supply, a container 130 and/or a pressure means 121.

The movement controller 111 is configured to generate the electric control signal for generating the translational oscillation movement of the cannula means 1 or the cannula 210 of the device for liposuction 200. The description of the electric control signal will not repeated for the sake of brevity. The movement controller 111 is powered preferably by a direct current voltage (VDC), preferably a VDC lower or equal than 36 VDC, preferably lower or equal than 24 VDC. The movement controller 111 could comprise a switching circuit, e.g. a pulse width modulation (PWM) to generate the electric control signal from the VDC. The movement controller 111 is preferably configured to control (meaning to change) the frequency and/or the amplitude of the electric control signal, preferably based on the user input from the user interface 112. The movement controller 111 can be configured to control two or more devices for liposuction 200 to work together on a patient or on two patients.

The control output 114 is configured for giving out the electric control signal to the device for liposuction 200. This is achieved by an electric connection 301, preferably a cable or a socket for plugging in a cable. However, it is also possible that the electric connection 301 is a wireless connection. However, this is less preferred as in this case, the device for liposuction 200 needs a power supply like a battery. In the latter case, the control output 114 is for example a radio communication means. If the movement controller 111 is configured to control two or more devices for liposuction 200, then the control output 114 comprises preferably two or more cables or sockets for connecting the control device 100 with the respective devices for liposuction 200.

The user interface 112 is configured to interact with the user, i.e. to receive user commands and/or to give out information to the user. The user interface 112 comprises preferably a user input means and/or a user output means. Preferably, the user input means is a display 112.1. Preferably, the display is a touch screen acting also as user input. Additionally or alternatively, other user inputs like buttons, knobs, mouse, keyboard, etc. are possible as user input means. Preferably, the user input means 112 comprises an interface for an external user input device like a pedal 112.2. The control device 100 or the movement controller 111 is preferably configured to control the on/off state of the device for liposuction and/or the amplitude or power of the oscillation movement based on the pedal 112.2. The control device 100 or the movement controller 111 is preferably configured to control the frequency of the oscillation movement based on the user input means, preferably based on the touch screen or some knobs. The display 112.1 displays preferably frequency and/or amplitude of the oscillation movement. If the movement controller 111 is configured to control two or more devices for liposuction 200, the user input means 112 comprises preferably two or more interfaces for external user input devices like pedals 112.2 to control the two or more devices for liposuction 200 independently.

The pressure means 121 is configured to create a pressure at the cannula 210 to extract substances/material, preferably fat from a body of a patient (via a depression) and/or to inject a substance, preferably physiologic liquid into the body of the patient. The pressure means 121 is connected via a fluid connection 304 to the container 130, the fluid connection 303 and/or the cannula 210 for creating the respective pressure at the cannula 210.

The pressure means 121 comprises a first pressure means 121.1 for creating a pressure (more precisely a depression) for drawing material/fat into the cannula 210 (and then through the fluid connection 303 into the container 130). The first pressure means 121.1 is preferably a vacuum pump. The pressure means 121, in particular the first pressure means 121.1 is configured to create a depression of more than -0.1 Bar, preferably than -0.3 Bar, preferably than -0.5 Bar, preferably than -0.7 Bar. The values given are relative pressure values with respect to the atmospheric pressure. The depression value is negative so that a an increasing depression value shall mean that the absolute depression value becomes larger. Preferably, the pressure means 121 is configured to control the pressure at the cannula 210. Preferably, the pressure means 121, in particular the first pressure means 121.1 is configured to control the pressure (or the depression), preferably between 0 Bar (no depression or atmospheric pressure) and a maximum depression value. The maximum depression value is preferably larger than -0.3 Bar, preferably than -0.5 Bar, preferably than -0.7 Bar. The maximum depression value is preferably smaller than -1.5 Bar, preferably than -1.2 Bar, preferably than -1 Bar. Preferably, the depression value is controlled based on a user input from the user input means 112. Preferably, the depression value is synchronized with the amplitude of the oscillation movement of the cannula 210 or of the cannula means 1. Preferably, the surgeon controls with the pedal 121.2 the amplitude of the oscillation movement and the depression value at the cannula 210 (and thus the suction power) together. If the movement controller 111 is configured to control two or more devices for liposuction 200, the pressure means 121, in particular the first pressure means 121.1 is configured to create a pressure, in particular a depression at the two or more cannulas 210 of the two or more devices for liposuction 210. Preferably, the pressure, in particular the depression at the two or more cannulas 210 of the two or more devices for liposuction 210 can be controlled by the pressure means 121 independently, preferably by the user input means 112, preferably by the respective pedals 112.2. This can be realized by two or more independent first pressure means 121.1 or by a common first pressure means 121.1 with two or more independent pressure controller.

In one embodiment, the pressure means 121 comprises a second pressure means 121.2 for creating a pressure (more precisely an overpressure) for injecting material/liquid out of the cannula 210 (from the container 130 and through the fluid connection 303). The second pressure means 121.2 can be used to inject physiological liquid into the body of the patient to facilitate subsequent fat removal from the body and/or to inject fat extracted previously from the patient back into his/her body. The injection of the material/liquid can be done concurrently with the oscillation movement of the cannula 210 as described for the extraction or without the oscillation movement. The first pressure means 121.1 and the second pressure means 121.2 can be realised as a combined depression/overpressure pump or could be realised as two distinct pumps.

The container 130 comprises at least one container for storing a substance extracted from a patient through the cannula 210 and/or for storing a substance to be injected into the body of the patient through the cannula 210. The substance extracted comprises preferably fat. The substance to be injected comprises preferably physiological liquid which can be injected before the fat removal operation to improve the removal of the fat from the remaining tissue of the body. The container 130 is connected or is connectable via the fluid connection 303 to the cannula 210 to move the extracted substance through the cannula 210, through the fluid connection 303 into the container 130 or to move the substance to be injected from the container 130 through the fluid connection 303 and through the cannula means 1 into the body of the patient. The container 130 or the fluid connection 210 is connected or is connectable via the fluid connection 303 and/or the fluid connection 304 to the pressure means 121 to create a pressure (more precisely a depression) which moves the extracted substance through the cannula 210, through the fluid connection 303 into the container 130 or to create a pressure (overpressure) to move the substance to be injected from the container 130, through the fluid connection 303, through the cannula 210 into the body of the patient. The container 130 is preferably connectable to the control device 100, e.g. by a container holder. The container is preferably disposable. The control device 100 can comprise one, two or more container 130 or could also comprise zero container 130, if an external container is used.. If the movement controller 111 is configured to control two or more devices for liposuction 200, the control device 100 comprises preferably two container 130 for extracted fat and/or two container holders for them.

The power supply is configured to provide electrical power for the control device 100 and/or for the device for liposuction 200. The power supply comprises preferably a socket or cable for a alternating current (AC) power grid for connecting the control device 100 to a AC power grid. The AC power grid has preferably an AC voltage of 110 V or 220 V. The power supply comprises preferably at least one power converter 113, 123 for converting the AC grid voltage into a DC supply voltage. The DC supply voltage is preferably lower or equal than 36 V, preferably lower or equal than 24 V, preferably lower or equal than 12 V. All items of the control device 100 are powered preferably by the supply voltage. The movement controller 111 is preferably configured to transfer the supply voltage into an electric control signal which powers the device for liposuction 200.

In a preferred embodiment, the control device 100 is constructed modularly. Preferably, the control device 100 comprises a modular housing for housing different modules depending on the modular configuration of the control device 100. The possible modules to be housed in the modular housing and/or the control device can comprises a movement control module 110 and/or a pressure module 120. Each module 110 and 120 can be removably connected to the modular housing in its entirety.

The movement control module 110 comprises preferably the movement controller 111 and the control output 114. Preferably, the movement control module 110 comprises further the user interface 112. However, in an alternative embodiment, it would also be possible to include the user interface 112 in a separate user interface module to be connectable as well to the modular housing. The control device 100 can preferably be configured between a first movement control module 110 and a second movement control module 110. The first movement control module 110 is configured to control (only) one device for liposuction 200 and/or comprises a control output 114 configured for connecting (only) one device for liposuction 200 and/or comprises a user interface 112 configured to connect (only) one external user input device 112.2, in particular (only) one pedal. The second movement control module 110 is configured to control two or more devices for liposuction 200 and/or comprises a control output 114 configured for connecting two (or more) devices for liposuction 200 and/or comprises a user interface 112 configured to connect two or more external user input devices 112.2, in particular two or more pedals. Preferably, the movement control module 110 comprises the power converter 113 receiving from the socket or cable 140 an AC grid voltage, when connected to the grid. The power converter 113 converts the AC grid voltage in the DC supply voltage of the movement control module 110.

The pressure module 120 comprises the pressure means 121 described above. Preferably, the pressure module 120 comprises also a pressure controller 122 for controlling the pressure module 120 and/or the pressure means 121. The pressure controller 122 is preferably connected to the movement module 110, the movement controller 111 and/or the user interface 112 for controlling the pressure of the pressure means. Preferably, the pressure means 121 is controlled over the user interface 112 of the movement controller and/or over the synchronization with the amplitude of the oscillation movement. In one embodiment, the pressure module 120 comprises a user interface (not shown) for controlling the pressure means 121, when there is no movement controller. In another embodiment, the pressure module has no independent user interface and cannot be controlled without the movement control module 110 or the user interface 112. Preferably, there exist different types of pressure modules 120. A first pressure module 120 comprises a pressure means 121, in particular a first pressure means 121.1 which is configured to create the pressure for (only) one device for liposuction 200. A second pressure module 120 comprises a pressure means 121, in particular a first pressure means 121.1 which is configured to create the pressure for two or more devices for liposuction 200. A third pressure module 120 corresponds to the first or second pressure module 120, but has additionally the second pressure means 121.2 for injecting material/liquid through the cannula 210 of the device for liposuction 200.

Preferably, the pressure module 120 comprises the power converter 123 receiving from the socket or cable 140 an AC grid voltage, when connected to the grid. The power converter 123 converts the AC grid voltage in the DC supply voltage of the movement control module 110. The independent power converters 113 and 123 in each module have the advantage that each module can be operated independently from the other. However, it would also be possible to provide the DC supply voltage from the movement control module 110 into the pressure module 120. However, this makes it more difficult to fulfil the strict electrical standards for medical devices. It would also be possible to have the power converter in the modular housing to provide the DC supply voltage directly to the modules 110, 120. In this case, the power converter could be also a module for itself which can be exchanged depending on the AC grid power provided. One module for a 110 V and one module for a 220 V grid and maybe other modules for further grid types.

The modular housing comprises preferably one, two or more fixtures for a container 130 or for a container holder. Thus, the control device 100 can be configured with zero, one, two or more container holders or containers 130 depending on the desired modular configuration.

The control device 100 can thus modularly be made out of a modular housing, one movement control module 110 and optionally one pressure module 120 and optionally one or more containers 130 or container holders. The movement controller 110 and/or the pressure module 120 can preferably be chosen from different types of movement modules 110 and/or from different types of pressure modules 120. The different types of movement modules include preferably the first movement controller 110 and the second movement controller 110. The different types of pressure modules include preferably the first pressure controller 120, the second pressure module 120 and/or the third movement controller 120. The control device 100 can thus be configured from a control device set comprising a modular housing, at least one, preferably at least two type(s) of movement control module(s) 110, at least one, preferably at least two type(s) of pressure module(s) 120. In a first configuration, the control device 100 comprise the first movement control module and no pressure module 120. In a second configuration, the control device 100 comprise the first movement control module 110 and the first pressure module 120. In a third configuration, the control device 100 comprise the first movement control module 110 and a third pressure module 120. Similar configurations are also possible with the second movement control module 110. Thus, the modular configuration of the control device 100 allows to manufacture easily many configurations of the control device 100 without the need to manufacture complete distinct control devices 100.

The described modular control device 100 is a preferred embodiment, but the control device 100 could also be arranged in a non-modular way. It is also possible to have the pieces of the control device 100 in different devices, e.g. one device corresponding to the movement control module 110 and a separate device corresponding to the pressure module 120.

In the system, the device for liposuction 200 is connected via the electric connection 301 to the control device 100, in particular the electric control output 114 is connected to the electric interface 4. The cannula 210 is connected to the cannula means 1 of the device for liposuction 200. The cannula 210 is connected via a fluid connection 303 and 304 to the container 130 and/or the pressure means 121.

The described system with a distinct control device 100 and a distinct device for liposuction 200 is preferred. However, it is also possible to have parts of the control device 100 in the device for liposuction 200, e.g. the movement controller 111, the power supply (maybe a battery), a user interface.

The described system is preferably used to extract material, preferably fat from the body of a patient. The cannula 210 is inserted into the relevant region of the body of the patient where the fat needs to be extracted. The surgeon selects the desired frequency and/or amplitude of the oscillation movement of the cannula 210 during the extraction process. Preferably, the frequency is controlled over the touch screen and/or the amplitude over a pedal. Preferably, the pressure means 121, in particular the first pressure means 121.1 is controlled to create a depression in the cannula 210 for drawing the fat from the body of the patient into the cannula 210. The fat is drawn through the fluid connection 303 into the container 130 by means of the depression created in the first pressure means 121.1. The pressure of the pressure means 121, in particular the depression of the first pressure means 121.1 is controlled preferably synchronized with the amplitude of the oscillation movement. Thus, if the surgeon presses the pedal 112.2, the cannula 210 starts with its oscillation movement with the frequency imposed by the user interface 112 and with an amplitude depending on the force received on the pedal 112.1. The pressure of the pressure means 121 increases at the same time depending on the force received on the pedal 1 12.1. Thus, the fat is extracted from the body of the patient by a depression of the first pressure means 121.1 drawing the fat into the cannula 210 while the cannula 210 performs the oscillation movement to help removing the fat from the body.

In a preferred embodiment, a physiological liquid is injected before the extraction of the fat. This is done by the same device for liposuction 210. The device for liposuction 210 could also be used to inject the extracted fat (maybe after a being cleaned or separated from other material) back into the body, preferably at another region. This can be done for aesthetic reasons or for improving a healing process. The injection is preferably done by creating a pressure, in particular an overpressure at the cannula 210 which pumps the material to be injected (physiological liquid/extracted fat) through the cannula 210 into the body. This injection can be performed with the oscillation movement of the cannula 210 or without.

The described embodiment was described with an electric control signal controlling the frequency and/or amplitude of the oscillation movement. In an alternative embodiment, it is also possible to control the frequency and/or amplitude by a different control input. The control input could be another control signal or even a mechanical input.

It should be understood that the present invention is not limited to the described embodiments and that variations can be applied without going outside of the scope of the claims.

## Claims

1. Device for liposuction comprising
a cannula means (1);
a movement means (2) configured to cause a translational oscillation movement of the cannula means (1) in a movement direction of the cannula means with a frequency smaller than 100 Hz;
**characterized in that**
the movement means (2) is configured to change the frequency and/or amplitude of the oscillation movement based on a control input.

2. Device according to the previous claim, the movement means (2) comprises an electrical actuator configured to transfer electrical energy into the translational oscillation movement of the cannula means (1).

3. Device according to the previous claim, wherein the movement means (2) is an electro-magnetic actuator configured to cause the translational oscillation movement of the cannula means (1) electro-magnetically.

4. Device according to the previous claim comprising a fixed portion and a movable portion, wherein the cannula means (1) is part of the movable portion, wherein the movable portion is supported by the fixed portion movably in the movement direction of the cannula means (1), wherein the movement means (2) comprises a first magnetic means in the fixed portion and a second magnetic means in the movable portion or connected to the movable portion, wherein one of the first and second magnetic means is an electro-magnetic means powered by electrical power and causing a magnetic field which interacts with the other of the first and second magnetic means to cause the translational oscillation movement of the movable portion and/or the cannula means (1).

5. Device according to the previous claim, wherein the other of the first and second magnetic means is a permanent magnet (22), wherein the electromagnetic means comprises two coils (21.1, 21.2) arranged in the movement direction at two sides of the permanent magnet (22).

6. Device according to one of the claims 2 to 5, wherein the electric actuator is electrically driven such that the movement of the movable portion is stopped electrically for moving in the opposed direction before stopping against the fixed portion.

7. Device according to one of the previous claims,
wherein the movement means (2) is configured to change the frequency between at least two different frequency values, wherein the at least two different frequency values comprise at least one frequency value between 10Hz and 25Hz,
wherein the movement means (2) is configured to change the amplitude between at least two different amplitude values, wherein the at least two different amplitude values comprise at least one full amplitude value between 3 and 15 mm.

8. Device according to one of the previous claims, wherein the control input is an electric control signal controlling the frequency and/or amplitude of the oscillation movement.

9. Device according to one of the previous claims being a handheld device.

10. Device according to one of the previous claims comprising a cannula (210) connected to the cannula means (1) or being part of the cannula means (1).

11. Control device for controlling a device for liposuction (200), the control device (200) comprising:
a movement controller (111) for generating a control signal for generating a translational oscillation movement of a cannula means (1) of the device for liposuction (200) with a frequency smaller than 100 Hz; and
a control output (114) for giving out the control signal to the device for liposuction (200);
**characterized in that** the movement controller (111) is configured to generate different control signals for generating the translational oscillation movement with different frequencies and/or amplitudes.

12. Control device according to claim 11 or 12, comprising a pressure (121) means connected to a cannula (210) of the device for liposuction (200) and configured :
to create a depression for drawing fat into the cannula and/or
to create a pressure for injecting an injection material out of the cannula (210) into a patient.

13. System for liposuction comprising:
a device for liposuction (200); and
a control device (100) for controlling the device for liposuction (200);
**characterized in that** the device for liposuction (200) is a device for liposuction (200) according to one of claims 1 to 10 and/or that the control device is a control device according to one of claims 11 to 12.

14. System according to the previous claim, comprising one or more of:
a cannula (210) connected to the device for liposuction (200);
at least one container (130) for storing extracted material and/or for storing material to be injected;
a fluid connection between the cannula (210) and the control device (100) and/or the at least container (130).

15. Process for operating a device for liposuction comprising the step of moving a cannula (210) translationally by an oscillation movement with a frequency smaller than 100 Hz;
**characterized in that**
the frequency and/or an amplitude of the oscillation movement is changeable based on a control input.
